# EUROPEAN PATENT APPLICATION

(11) **EP 1 890 157 A1**
(43) Date of publication of application: **20.02.2008**
(21) Application number: 06756441.9
(22) Date of filing: 22.05.2006
(51) Int. Cl.: G01N 35/10, B03C 1/00, G01N 33/553, C12M 1/00

(54) **METHOD FOR REPLACING LIQUID, METHOD FOR EXTRACTING COMPONENT BY USING IT, COMPOSITE CONTAINER AND AUTOMATIC ANALYZER**

(30) Priority: 07.06.2005 JP 2005166775
(71) Applicant: Arkray, Inc., Kyoto-shi, Kyoto 601-8045 (JP)
(72) Inventor: IZUMIZAWA, Yuji c/o Arkray, Inc., Kyoto-shi Kyoto 601-8045 (JP)
(74) Representative: Neath, Susannah Mairi
(86) International application number: PCT/JP2006/310135
(87) International publication number: WO 2006/132074

(57) **Abstract**

A liquid exchange method of exchanging liquids present around a magnetic substance is provided. The method serves to prevent a remaining liquid from transferring to a next step and a loss of the magnetic substance. Even when the liquid exchange method is applied to an autoanalyzer, the device structure will not be complicated. A vessel 11 in which a liquid 16 containing a magnetic substance 15, a vessel 12 containing a liquid 17, a magnetic substance move-mediating member 13 and also a magnet 18 are prepared. The magnet 18 is disposed at the exterior of the vessel 11, and the magnet 18 is moved to move the magnetic substance 15 on the inner wall face of the vessel 11 in order to take out the magnetic substance 15 from the magnetic substance containing liquid 16, to move the magnetic substance 15 from the inner wall face of the vessel 11 to the surface of the magnetic substance move-mediating member 13, and to move to the inner wall face of the other vessel 12 via the magnetic substance move-mediating member 13 so as to introduce the magnetic substance 15 into the liquid 17 in the vessel 12, thereby exchanging the liquids present around the magnetic substance 15.

## Description

### Technical Field

The present invention relates to a liquid exchange method of exchanging liquids present around a magnetic substance, an ingredient extraction method using the liquid exchange method, a composite container and an autoanalyzer.

### Background Art

In the fields of biology and chemistry, magnetic substances have been used conventionally as a solid phase for binding particular materials so as to extract or separate the particular materials for example. Specific examples thereof include extraction or separation of cells, microbes, viruses, protein, nucleic acids and the like.

When such a magnetic substance is used, processes such as centrifugation and filtration can be omitted. For example, by using the nature of a nucleic acid to be adsorbed by silica in the presence of a chaotropic agent and also by using a magnetic substance, the nucleic acid can be extracted from cells in the following manner. First, magnetic fine particles (magnetic beads) whose surfaces are coated with silica are prepared. In a test tube, a solution containing disrupted cells and an adsorption solution (a solution containing the chaotropic agent and a protein denaturant) are mixed with the magnetic fine particles so that the nucleic acid is adsorbed onto the surfaces of the magnetic fine particles. Subsequently, a magnet is disposed outside the bottom of the test tube so as to attract the magnetic fine particles in the test tube and to hold and fix the magnetic fine particles on the bottom of the test tube. In this state, by using a pipette or the like, the adsorption solution in the test tube is aspirated and a washing liquid is injected in place of the adsorption solution in the test tube so as to wash the inner wall of the test tube and the magnetic fine particles. After repeating this washing operation two or three times, the washing liquid is discharged. Further, an elution liquid (distilled water) is injected into the test tube so that the nucleic acid adsorbed onto the surfaces of the magnetic fine particles is transferred into the elution liquid. By recovering the elution liquid, the nucleic acid can be extracted.

In such operations including extraction and separation of a particular material by using a magnetic substance, a basic operation includes an exchange of liquids present around the magnetic substance. However, conventional liquid exchange methods may cause problems as mentioned below. First, in a liquid exchange in the nucleic acid extraction method, the magnetic fine particles are collected on the bottom of the test tube as mentioned above, and then discharged by aspirating the liquid in the test tube. However, it is difficult to discharge the liquid completely. Moreover, since the liquid exchange is carried out in the same test tube, the remaining liquid may be included in the following step. Furthermore in this method, when the liquid in the test tube is aspirated vigorously to discharge the liquid completely, the magnetic fine particles may be aspirated and discharged as well.

In a method for solving the problems, for example, the magnetic fine particles are held and fixed in a liquid aspiration line that is used for discharging the liquid from the test tube (Patent document 1). According to this method, the liquids are not exchanged in the same test tube but the next step is carried out afresh in another test tube, and thus the remaining liquid will not be included in the next step. However in this method, the liquid and the magnetic fine particles in the test tube may not be aspirated completely. Moreover, as the magnetic fine particles are held and fixed in the aspiration line, at the time of discharging a waste liquid, the magnetic fine particles may be discharged as well due to the discharging power. This may cause the problem of loss of the magnetic fine particles. In another liquid exchange method, a covered magnet is introduced directly into a liquid containing magnetic fine particles in a test tube so that the magnetic fine particles are adsorbed. The magnet is moved into a liquid in another test tube, and the magnetic force is shielded or extinguished so as to transfer the magnetic fine particles into a liquid in the other test tube and to exchange the liquids (Patent documents 2, 3). According to this method, the loss of the magnetic fine particles can be reduced and the problem of transferring of the remaining liquid into the next step can be solved. In this method, however, the magnet must be disposed right above the test tube. Therefore, when this method is applied to an autoanalyzer, the magnet will be an obstacle for an aspiration-discharge device or an agitator that must be disposed similarly right above the test tube. As a result, the device structure will be complicated.
Patent document 1: JP 3115501
Patent document 2: JP 2727075
Patent document 3: JP 3254681

### Disclosure of Invention

### Problem to be Solved by the Invention

Therefore, with the foregoing in mind, it is an object of the present invention to provide a liquid exchange method for exchanging liquids present around a magnetic substance, which can prevent transferring of a remaining liquid into a next step and a loss of the magnetic substance. Even when the liquid exchange method is applied to an autoanalyzer, the device structure will not be complicated.

### Means for Solving Problem

For achieving the above-described object, a liquid exchange method of the present invention is a liquid exchange method for exchanging liquids present around a magnetic substance, and the method includes: providing a plurality of vessels containing liquids, a member for mediating a movement of the magnetic substance from one vessel to another vessel (hereinafter, referred to as "magnetic substance move-mediating member"), and a magnet, where the liquid in at least one of the vessels is a magnetic substance containing liquid; disposing the magnet at the exterior of the vessel; moving the magnet so as to move the magnetic substance in the magnetic substance containing liquid on the inner wall face of the vessel in order to take out the magnetic substance from the magnetic substance containing liquid; moving the magnet so as to move the magnetic substance from the inner wall face of the vessel to the surface of the magnetic substance move-mediating member; moving the magnet so as to move the magnetic substance to an inner wall face of another vessel via the surface of the magnetic substance move-mediating member in order to introduce the magnetic substance into a liquid in the other vessel, thereby exchanging liquids present around the magnetic substance.

### Effects of the Invention

Therefore in the liquid exchange method of the present invention, a liquid exchange is carried out by moving the magnetic substance into plural vessels without a direct adsorption of the magnetic substance by a magnet. Therefore, unlike the conventional techniques, a remaining liquid can be prevented from being included in the next step, and also the loss of the magnetic substance can be prevented. Further, in the liquid exchange method of the present invention, since there is no necessity of disposing the magnet right above the vessel, the device structure will not be complicated even when the method is applied to an autoanalyzer.

### Brief Description of Drawings

[FIG. 1] FIG. 1 includes views showing an example of a composite container used for a liquid exchange method of the present invention. FIG. 1(A) is a perspective view, FIG. 1(B) is a front view, FIG. 1(C) is a back view, and FIGs. 1(D) and 1(E) are cross-sectional views.
[FIG. 2] FIG. 2 includes process diagrams showing an example of a liquid exchange method of the present invention. FIGs. 2(A-1) to 2(A-4) are cross-sectional views and FIG. 2(B-1) to 2(B-4) are back views.
[FIG. 3] FIG. 3 includes views showing an example of a cartridge used for a liquid exchange method of the present invention. FIG. 3(A) and 3(B) are perspective views and FIG. 3(C) is a cross-sectional view.
[FIG. 4] FIG. 4 is a cross sectional view showing an injection of a reagent liquid in the cartridge.
[FIG. 5] FIG. 5 is a cross-sectional view showing a liquid exchange in the cartridge.
[FIG. 6] FIGs. 6(A)-(F) are cross-sectional views showing variations of liquid exchange vessels in a cartridge used for the liquid exchange method of the present invention.
[FIG. 7] FIG. 7 is a perspective view showing a variation of a cartridge used for a liquid exchange method of the present invention.
[FIG. 8] FIG. 8 is a graph showing a result of an extraction method of the present invention.

### Explanation of letters and numerals

8: bottomed cylinder
11,12,31,32,33,34,35,81,82,83,84,85,86: liquid exchange vessel
13,61,88: magnetic substance move-mediating member
14: horizontal plate
15: magnetic substance
16,17: liquid
18,87: magnet
21,22,23,24,25: reagent vessel
41: sealing member
62: plate
63: liquid aspiration-discharge system
64: holding part
*a,b,c,d,e*: reagent liquid

### Description of the Invention

It is preferable in the liquid exchange method of the present invention, that, in a case where the magnetic substance is introduced into another (second) vessel, the magnet is moved to move the magnetic substance on the inner wall face of the second vessel into the liquid. Accordingly, the magnetic substance can be transferred with certainty into the liquid of the second vessel. However, the present invention is not limited to this method. In an alternative method, after moving onto the inner wall face of the second vessel, the magnetic force of the magnet is shielded or extinguished above the liquid in the second vessel so that the magnetic substance drops naturally to be transferred into the liquid.

Furthermore in the liquid exchange method of the present invention, it is possible to move the magnet so as to move the magnetic substance on the inner wall face of the second vessel in order to take out the magnetic substance from the magnetic substance containing liquid, to move the magnet so as to move the magnetic substance from the inner wall face of the second vessel to the surface of the magnetic substance move-mediating member, to move the magnet so as to move the magnetic substance onto an inner wall face of a still another (third) vessel via the surface of the magnetic substance move-mediating member in order to introduce the magnetic substance into the liquid in the third vessel. This series of operations can be repeated.

In the present invention, the type of the magnetic substance is not limited particularly, but the examples include iron, ferric oxide (e.g., Fe₃O₄ (magnetite), Fe₂O₃ (maghemite) and the like), chromium, chromium oxide, nickel, an magnetic alloy and the like. For these magnetic substances, for example, commercially-available products can be used. Among the above-mentioned magnetic substances, iron and ferric oxide are preferred. Ferric oxide is preferred further. Alternatively, the magnetic substance can be prepared by combining any of the above-mentioned magnetic substances and a non-magnetic substance. Though the shape of the magnetic substance is not limited particularly in the present invention, the examples include magnetic fine particles (magnetic beads), and aggregates of magnetic fine particles. Though the size of the magnetic fine particles is not limited particularly, when the magnetic fine particles are spherical, the diameter (average particle diameter) is in a range of 0.01 to 100 µm for example, preferably 0.1 to 50 µm and more preferably 0.1 to 10 µm.

The surfaces of the magnetic fine particles can be coated with various materials. For example, for extraction of a nucleic acid, magnetic fine particles coated with silica can be used. Since the coating on the magnetic fine particles is extremely thin, the size of the coated magnetic fine particles does not change substantially

Though there is no particular limitation for the magnet in the present invention, for example, permanent magnets and electromagnets or the like can be used. The magnetic force of the magnet is not limited particularly as long as the magnet can move the magnetic substance.

Next, the present invention refers to an ingredient extraction method of extracting an ingredient from a sample, and the method includes an ingredient extraction step of adsorbing an extraction target ingredient onto a magnetic substance in an adsorption solution; exchanging in this state the adsorption solution for a washing liquid; washing and removing ingredients other than the extraction target ingredient; and further exchanging the washing liquid for an elution liquid so as to elute the extraction target ingredient in the sample, where the exchange of the liquids in the ingredient extraction step is carried out by the liquid exchange method of the present invention.

The extraction target ingredient is not limited particularly, and the examples include nucleic acids, protein, sugar, lipid, viruses, microbes, antibodies, antigens and the like. The sample is not limited particularly, and the examples include: a sample of biological origin; an environmental sample obtained from domestic waste water, industrial liquid waste and the like; and chemical samples used for a chemical analysis. Examples of the sample of biological origin include whole blood, lymph, urine, saliva, sputum, nasal secretion, cells, biomedical tissues, organs, microbes, viruses and the like. An example of the cells is a cell culture, and examples of the cell culture include an animal cell culture, a plant cell culture, a microbial culture and the like.

In the ingredient extraction method according to the present invention, the magnetic substance is not limited particularly as long as it can adsorb the extraction target ingredient, and materials as described above can be used. For example, magnetic substances coated with various materials can be used in accordance with the types of the extraction target ingredients. Specifically, a magnetic substance coated with a material to adsorb the extraction target ingredient can be used. For example, as mentioned above, silica is used for the coating material when the target ingredient is a nucleic acid. An antibody can be used for the case of a virus, sugar can be used for the case of protein, and cellulose or the like is used for the case of lipid. For materials other than these examples, well-known magnetic substances and coated magnetic substances can be used.

Next, the present invention refers to a composite container used in the liquid exchange method of the present invention or the ingredient extraction method of the present invention. The composite container has a plurality of vessels and a magnetic substance move-mediating member, and the plural vessels and the magnetic substance move-mediating member are integrated with each other.

It is preferable that the composite container has further a reagent vessel containing a reagent liquid. It is preferable also that the composite container is a cartridge for an autoanalyzer.

Next, the present invention refers to an autoanalyzer for analyzing an ingredient in a sample. The autoanalyzer has an ingredient extraction member for extracting an ingredient from the sample, and the ingredient extraction member denotes a means for extracting an ingredient according to the ingredient extraction method of the present invention.

It is preferable that the autoanalyzer of the present invention further has a magnet moving member, and the magnet is moved by the magnet moving member.

It is preferable in the autoanalyzer of the present invention that the composite container of the present invention is used. In this case, it is preferable that the autoanalyzer of the present invention further has a composite container moving member, with which the composite container is moved so as to move the magnetic substance relatively.

Next, examples of the present invention will be described below with reference to the attached figures.

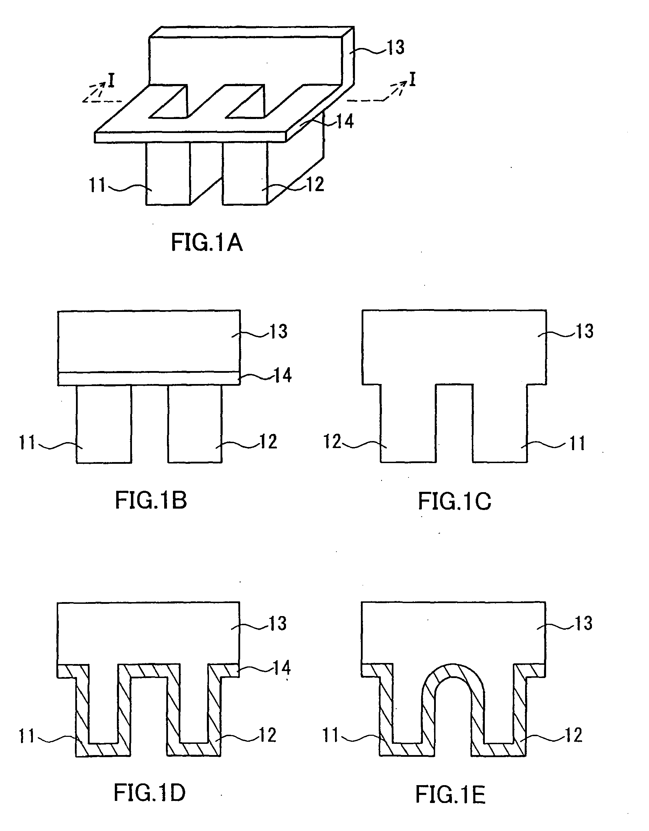

The liquid exchange method of the present invention is performed by using the plural vessels, the magnetic substance move-mediating member, and the magnet. FIG. 1 shows an example of a composite container formed by integrating the plural vessels and the magnetic substance move-mediating member. FIG. 1(A) is a perspective view showing the composite container, FIG. 1(B) is the front view, FIG. 1(C) is the back view, FIG. 1(D) is a cross-sectional view taken along the line I-I in FIG. 1(A), and FIG. 1(E) is a cross-sectional view showing a variation of the composite container. In these figures, the same components are assigned with the same reference signs.

As shown in the figures, the composite container has two liquid exchange vessels 11, 12. Openings of the vessels are linked by a linking member that is formed by linking a horizontal plate 14 and a vertical plate 13 (the magnetic substance move-mediating member 13) and that has a substantially L-shape in the cross section, and the three components are integrated with each other. This composite container has a flat and smooth back face so that the magnet will move easily. As shown in the cross-sectional view of FIG. 1(D), the spacing (junction) of the vessels can be made angular. Alternatively, it can be a curve (arch shape) as shown in the cross-sectional view of FIG. 1(E). With the curve (arch shape), the magnetic substance can be moved smoothly. In this composite container, the magnetic substance move-mediating member 13 links the inner wall face of the liquid exchange vessel 11 and the inner wall face of the other liquid exchange vessel 12.

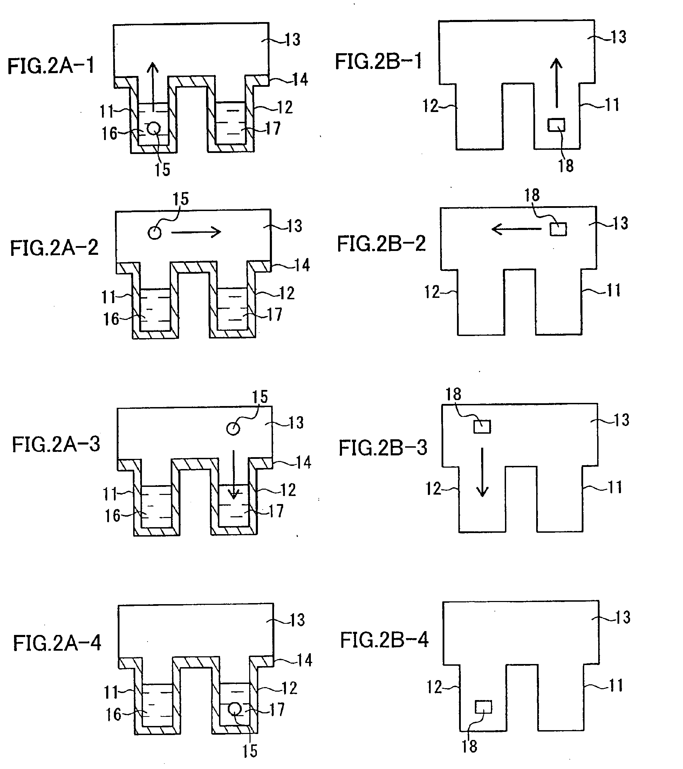

A liquid exchange by use of the composite container and the magnet is carried out as shown in FIG. 2 for example. FIGs. 2(A-1) to 2(A-4) are cross-sectional views (cross-sectional views taken along the line I-I in FIG. 1(A)) showing the steps of the liquid exchange when viewed from the front of the composite container. FIGs. 2(B-1) to 2(B-4) are back views showing the steps of the liquid exchange when viewed from the back face of the composite container. In these figures, the same components as those of FIG. 1 are assigned the same reference signs.

First, as shown in FIG. 2(A-1), the vessel 11 in the composite container contains a liquid 16 in which a magnetic substance 15 is present, and the vessel 12 contains another liquid 17. And as shown in FIG. 2(B-1), a magnet 18 is disposed on the back face of the vessel 11 so as to hold the magnetic substance 15 on the inner wall face of the vessel 11. Next, as shown in FIGs. 2(A-1) and 2(B-1), the magnet 18 is moved upwards so as to take the magnetic substance 15 out from the liquid 16 in order to move the magnetic substance onto the surface of the magnetic substance move-mediating member 13. Next, as shown in FIGs. 2(A-2) and 2(B-2), the magnet 18 is moved horizontally (a direction designated with an arrow in FIG. 2(B-2)) so as to move the magnetic substance 15 horizontally (a direction pointed with an arrow in FIG. 2(A-2)) on the surface of the magnetic substance move-mediating member 13 up to the position right above the vessel 12. Next, as shown in FIGs. 2(A-3) and 2(B-3), the magnet 18 is moved downwards to move the magnetic substance 15 into the liquid 17 in the vessel 12. In this manner, the liquids present around the magnetic substance 15 can be exchanged.

In a case where the junction between the vessel 11 and the vessel 12 forms a curve (arch shape) as shown in FIG. 1(E), the magnet can be moved not on the back face of the composite container but on the inner side wall face of the vessel 11 (the right side wall face in this figure), the lower outer wall face of the curve (arch shape) junction, and on the inner side wall face of the vessel 12 (the left side wall face in this figure). In such a case, the curve (arch shape) junction between the vessels on the horizontal plate 14 will form the magnetic substance move-mediating member.

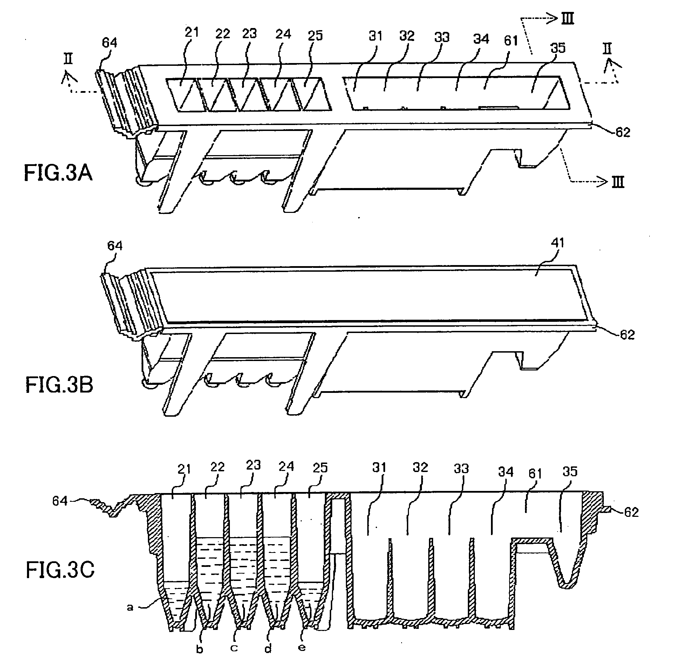

Next, the cartridge for the autoanalyzer used in the liquid exchange method of the present invention will be described with reference to the attached figures. FIG. 3 shows an example of the cartridge of the present invention. FIG. 3(A) is a perspective view showing a state where the top of the cartridge is not sealed. FIG. 3(B) is a perspective view showing a state where the top of the cartridge is sealed. FIG. 3(C) is a cross-sectional view taken along the line II-II of FIG. 3(A). In these figures, the same components are assigned with the same reference signs.

As shown in the figures, this cartridge is formed by disposing serially, below a plate 62 having a rectangular plane, five reagent vessels (21, 22, 23, 24, 25) and five liquid exchange vessels (31, 32, 33, 34, 35) that are integrated with each other. The half of the rectangular plate 62 (left side in the figure) has a flat and smooth surface, in which openings of the five reagent vessels (21, 22, 23, 24, 25) are positioned. The other half part of the rectangular plate 62 has a rectangular depression as a concave, and the five liquid exchange vessels (31, 32, 33, 34, 35) are positioned at the lower part of the concave. The five reagent vessels (21, 22, 23, 24, 25) are shaped as angular tubes being polygonal in lateral cross sections and having tapered bottoms, in which a reagent liquid *a,* a reagent liquid *b*, a reagent liquid *c*, a reagent liquid *d* and a reagent liquid e are contained respectively. Among the five liquid exchange vessels (31, 32, 33, 34, 35), the four liquid exchange vessels (31, 32, 33, 34) closer to the reagent vessel side are shaped as bottomed angular tubes adjacent to each other. The remaining one liquid exchange vessel 35 is shaped as an angular tube polygonal in a lateral cross section and having a tapered bottom, and spaced from the other liquid exchange vessels. As the liquid exchange vessel 35 is separated in this manner, when the liquid exchange vessel 35 is heated, the heat is prevented from being transferred to the remaining liquid exchange vessels. These liquid exchange vessels (31, 32, 33, 34, 35) are empty in a pre-use state. The side wall of the concave of this cartridge forms the magnetic substance move-mediating member 61, and one end part (the left end part in the figure) of the plate 62 forms a holding part 64. As shown in FIG. 3(B), in the cartridge of this example in a pre-use state, a sealing member 41 is adhered on the upper face of the plate 62 so as to cover the openings of the five reagent vessels (21, 22, 23, 24, 25) and the five liquid exchange vessels (31, 32, 33, 34, 35).

The size of the cartridge of the present invention is not limited particularly and it is determined suitably in accordance with the numbers, sizes and the like of the reagent vessels and the liquid exchange vessels. For example, the size of the cartridge as a whole is as follows. For example, the maximum length in the longitudinal direction is in a range of 30 to 250 mm, the maximum length in the width direction is in a range of 5 to 20 mm, and the maximum height is in a range of 5 to 100 mm. In the cartridge, the sizes of the reagent vessels and the liquid exchange vessels are not limited particularly, and can be determined suitably in accordance with the amount of the samples to be processed and the amount of the reagents. For example, the height is in a range of 5 to 100 mm, the maximum inner diameter is in a range of 5 to 20 mm, the longitudinal length is in a range of 5 to 20 mm, and the transverse length is in a range of 5 to 20 mm. In the cartridge of the present invention, the number of the vessels is not limited particularly. For example, in the cartridge, the number of the reagent vessels is in a range of 1 to 10 for example, and the number of the liquid exchange vessels is in a range of 1 to 10 for example, and preferably 2 to 10.

There is no particular limitation for the materials of the cartridge of the present invention, and polystyrene, polyethylene and polypropylene can be used for example. Similarly to the case of the above-described cartridge, it is preferable in the cartridge of the present invention that the openings of vessels are sealed with a sealing member. Examples of the materials for the sealing member include plastic films of polyethylene, vinyl and the like, an aluminum foil, and an aluminum foil laminated with a thermoplastic resin. The sealing member is stuck on the upper face of the plate 62 with a hot-melt adhesive or the like for example. The cartridge of the present invention also can have a vessel for another purpose such as a liquid waste recovery and a chip recovery, in addition to the reagent vessels and the liquid exchange vessels. When a part of the cartridge of the present invention forms an analyzing part for performing an analysis by means of an optical process, the analyzing part is transparent preferably. Such a transparent analyzing part can be formed with a transparent plastic like polystyrene for example.

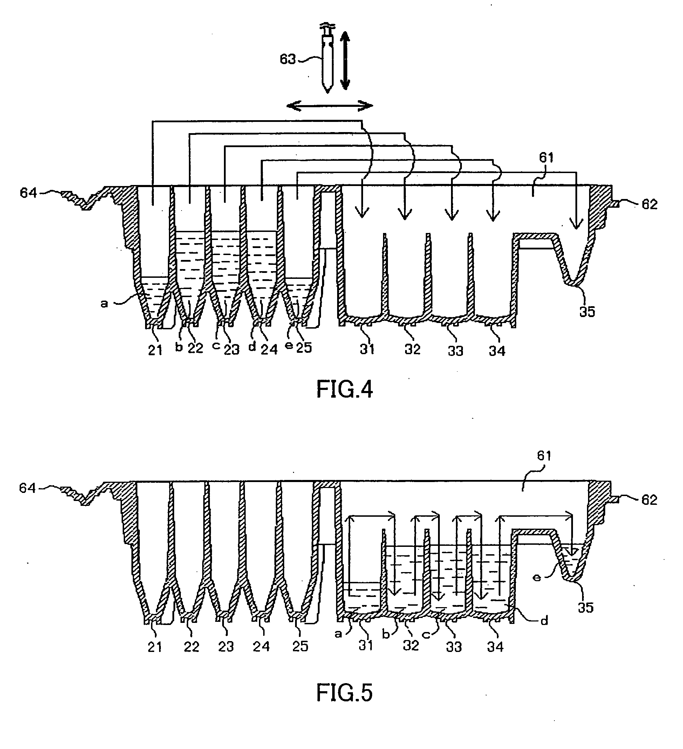

The use of the above-mentioned cartridge as shown in FIG. 3 will be described with reference to the cross-sectional views of FIG. 4 and FIG. 5 regarding extraction of a nucleic acid for example. In these figures, the same components as those in FIG. 3 are assigned the same reference signs.

Namely, first, the cartridge with the five reagent vessels (21, 22, 23, 24, 25) is prepared. The reagent vessels contain respectively an extraction solution (containing a chaotropic agent and silica-coated magnetic fine particles) for the reagent liquid *a*, a first washing liquid (containing a protein denaturant) for the reagent liquid *b*, a second washing liquid (containing an organic solvent such as ethanol) for the reagent liquid *c*, a third washing liquid (containing an organic solvent such as ethanol) for the reagent liquid *d*, and an elution liquid (distilled water) for the reagent liquid *e*. The cartridge is set in an autoanalyzer. In the cartridge, all the five liquid exchange vessels (31, 32, 33, 34, 35) are empty.

Next, as shown in FIG. 4, by using the liquid aspiration-discharge system 63 of the autoanalyzer, the respective reagent liquids (*a, b, c, d, e*) in the respective reagent vessels (21, 22, 23, 24, 25) are transferred to the liquid exchange vessels (31, 32, 33, 34, 35). In this figure, the respective arrows indicate the transferring of the respective reagent liquids (*a*, *b, c, d, e*). By the time of this transferring, the sealing member 41 stuck on the upper face of the plate 62 has been removed with a seal breaker or the like. Before/in the midst/after the transferring of the respective reagent liquids, samples of biological origin such as a solution of disrupted cells or a whole blood are introduced into the liquid exchange vessel 31.

Next, as shown in FIG. 5, the magnetic substance is moved to extract the nucleic acid from the sample of biological origin. That is, first, in the extraction solution (reagent liquid *a*) in the liquid exchange vessel 31, the nucleic acid is adsorbed onto the surface of the magnetic substance (not shown). Then, as indicated with an arrow in this figure, the magnetic substance is held on the wall face of the liquid exchange vessel 31 by a magnet (not shown), and by moving the magnet, the magnetic substance is introduced into the first washing liquid (reagent liquid *b*) in the next liquid exchange vessel 32 via the surface of the magnetic substance move-mediating member 61 and subjected to a first washing step therein. During this first washing, the magnetic force of the magnet is shielded or extinguished for example so as to disperse the magnetic substance sufficiently in the first washing liquid. At this time, an agitator can be used for a forcible dispersion.

Next, by using the magnet, the magnetic substance is taken out from the first washing liquid (reagent liquid *b*) in the liquid exchange vessel 32 and introduced into the second washing liquid (reagent liquid *c*) in the next liquid exchange vessel 33 via the surface of the magnetic substance move-mediating member 61 as indicated with an arrow so as to be subjected to a second washing as mentioned above. Next, by using the magnet, the magnetic substance is taken out from the second washing liquid (reagent liquid *c*) in the liquid exchange vessel 33 and introduced into the third washing liquid (reagent liquid *d*) in the next liquid exchange vessel 34 via the surface of the magnetic substance move-mediating member 61 as indicated with an arrow. Next, by using the magnet, the magnetic substance is taken out from the third washing liquid (reagent liquid *d*) in the liquid exchange vessel 34 and introduced into the elution liquid (reagent liquid e) in the next liquid exchange vessel 35 via the surface of the magnetic substance move-mediating member 61 as indicated with an arrow. Here, as mentioned above, the magnetic substance is dispersed in the elution liquid to transfer the nucleic acid into the elution liquid. At this time, it is possible to heat the liquid exchange vessel 35 so as to accelerate the extraction of the nucleic acid. In this manner, the nucleic acid is extracted into the elution liquid. The nucleic acid ingredient can be analyzed by introducing an analytic reagent or the like in the liquid exchange vessel 35. Alternatively, it is possible to recover the elution liquid for the purpose of analysis with an analytic reagent or the like in another analytic vessel.

In the present invention, the method for moving the magnet is not limited particularly. It is possible for example that the above-described cartridge is fixed and the magnet is moved by using a driving device in an order as indicated with the arrows in FIG. 5. Alternatively, it is possible to fix the magnet and move the cartridge itself so as to move the magnetic substance relatively as indicated with the arrows in FIG. 5. Alternatively in the cartridge, it is possible to move the magnet vertically and move the cartridge horizontally with the driving device so as to move the magnetic substance as indicated with the arrows in FIG. 5.

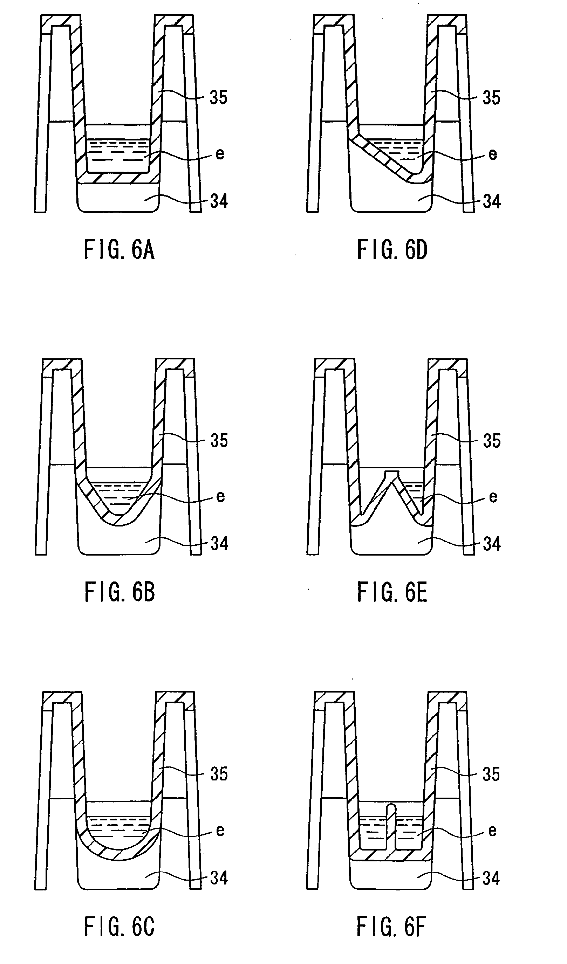

The liquid exchange vessel 35 for the final process in the cartridge in FIG. 3 is shown as an example in FIG. 6. FIG. 6(A)-6(F) are cross-sectional views taken along the line III-III in FIG. 3(A). In these figures, the same components are assigned with the same reference signs. Regarding the liquid exchange vessel 35 in FIG. 6, the face on which the magnet can move as shown in FIGs. 3-5 is regarded as 'side face'.

According to a first embodiment of the liquid exchange vessel, as shown in FIG. 6(A), the cross-section is shaped as a concave, and the two side faces are disposed vertically. In the cross section viewed from the side face, the bottom end part is tapered as shown in FIG. 3(C). As the two side faces are disposed vertically in this manner, the magnet can be moved easily. Moreover, since the side faces themselves are tapered, the capacity of the liquid exchange vessel 35 can be reduced. In a second embodiment of the liquid exchange vessel, as shown in FIG. 6(B), not only the cross section viewed from the side face but the cross section taken along the line III-III in FIG. 3(A) are tapered, and thus the liquid exchange vessel is shaped overall as a quadrangular pyramid. With this embodiment, the capacity can be reduced further. Moreover, by moving the magnet along the side faces from the vertical plane to the oblique plane, it is possible to move the magnetic substance to the tip end part of the liquid exchange vessel 35. Since the magnetic force applied to the magnetic substance is weakened gradually from the oblique part when the magnet moves only in the vertical direction, it is possible to drop the magnetic substance in the reagent at the bottom of the liquid exchange vessel 35 automatically. In a third embodiment of the liquid exchange vessel, as shown in FIG. 6(C), the tip end part of the liquid exchange vessel 35 forms a curve (arch shape). According to this embodiment, the capacity can be reduced further, and the magnetic substance can be moved smoothly. In a fourth embodiment of the liquid exchange vessel, as shown in FIG. 6(D), one side face forms a vertical plane, and the other side face has an oblique plane. According to this embodiment, the magnetic substance can be moved easily on the side face as the vertical plane, and the capacity can be reduced by making the other side face as an oblique plane.

In a fifth embodiment of the liquid exchange vessel, as shown in FIG. 6(E), two side faces are disposed vertically just like in FIG. 6(A), and a convex is provided on the bottom so as to divide the interior to form two wells. In general, one magnet must be provided for each cartridge. However, by arranging two cartridges so that the respective side faces correspond to each other and by disposing a magnet between the cartridges, one magnet can move magnetic substances in two cartridges respectively at the same time. In this case, since it is desired that the one magnet exhibit magnetic force of substantially the same level on the side faces of the two cartridges, it is preferable that the side faces of the cartridges facing the magnet are vertical planes. Furthermore, it is preferable that both of the two side faces of the cartridge are vertical planes so that there is no necessity of determining in use which of the two cartridges should be disposed at the right/left of the magnet. In such a case, according to the liquid exchange vessel in FIG. 6(E), since both the side faces are vertical planes, even when two cartridges are arranged, one magnet can move both the magnetic substances at the same time. Furthermore, since the interior is divided into two wells, any of the wells can be used. Namely, in use, a well having the side face provided with the magnet can be selected, and the capacity can be reduced. A sixth embodiment of the liquid exchange vessel as shown in FIG. 6(F) is a variation of the liquid exchange vessel in FIG. 6(E). The interior is divided into two wells by providing a partition.

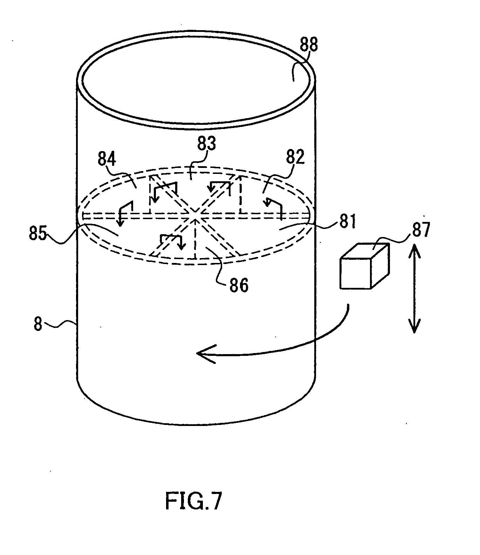

In the present invention, the shape of the cartridge is not limited particularly. In addition to the shape as shown in FIG. 3, for example, it is possible to partition the interior of a bottomed cylindrical vessel 8 so as to form plural (six) vessels (81, 82, 83, 84, 85, 86) as shown in the perspective view of FIG. 7. In this cartridge, the six vessels (81, 82, 83, 84, 85, 86) form liquid exchange vessels, and the cylindrical container inner wall part above them forms a magnetic substance move-mediating member 88. In this cartridge, as indicated with the arrows, the cylindrical container 8 is rotated in the circumferential direction and at the same time, the magnet 87 is moved vertically so as to move the magnetic substance (not shown) from the liquid in one of the vessels to the liquid in another vessel via the surface of the magnetic substance move-mediating member 88.

Next, Example of the present invention will be described below together with Comparative Example. It should be noted that the present invention will not be limited by the Example and the Comparative Example below.

### Example 1

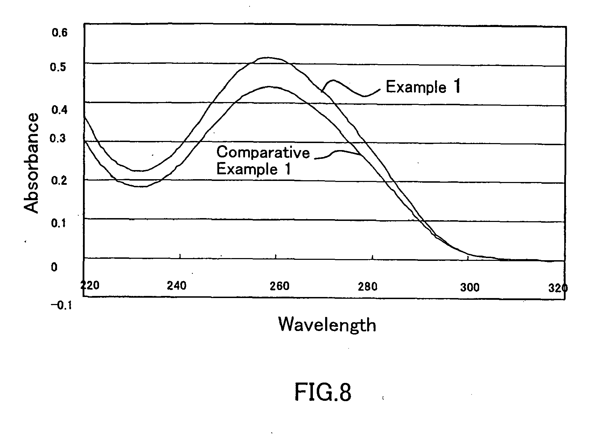

The reagent liquids *a*-*e* (reagent liquid MagExtractor (registered trade name) manufactured by TOYOBO CO., LTD.) were injected into the five liquid exchange vessels (31, 32, 33, 34, 35) of the cartridge as shown in FIG. 3 in the above-mentioned manner. And 100 µL of blood (whole blood) was added to the liquid exchange vessel 31 of the cartridge. After mixing sufficiently, the magnetic substance was moved sequentially among the liquid exchange vessels (31, 32, 33, 34, 35) by using a magnet for exchanging the liquids, and thus a nucleic acid in the blood was extracted. Absorbances of the reagent liquid *e* at 260 nm (absorption wavelength of nucleic acid) and at 280 nm (absorption wavelength of protein) were measured with a spectrophotometer so as to calculate a nucleic acid purification degree (260 nm absorbance / 280 nm absorbance) and the nucleic acid concentration (recovery). The results are shown in Table 1 below and the graph of FIG. 8. When the nucleic acid purification degree is in a range of 1.8-1.9, it is determined as excellent.

Reagent liquid *a*: 750 µL of dissolution adsorption solution + 40 µL of magnetic bead solution
Reagent liquid *b*: 900 µL of first washing liquid in the MagExtractor (registered trade name)
Reagent liquid *c*: 900 µL of second washing liquid in the MagExtractor (registered trade name)
Reagent liquid *d*: 900 µL of 70% ethanol solution
Reagent liquid *e*: 100 µL of extraction solution (corresponding to elution liquid: sterilized water)

### (Operation 1)

The reagent liquid *a* (750 µL of dissolution adsorption solution + 40 µL of magnetic bead solution) was injected into the liquid exchange vessel 31 in FIG. 3. The reagent liquid *b* (900 µL of the first washing liquid) was injected into the liquid exchange vessel 32, the reagent liquid *c* (900 µL of the second washing liquid) was injected into the liquid exchange vessel 33, the reagent liquid *d* (900 µL of 70% ethanol) was injected into the liquid exchange vessel 34, and the reagent liquid e (100 µL of sterilized water) was injected into the liquid exchange vessel 35 respectively. The magnetic beads denote beads having an average particle diameter of 1 to 10 µm (coated with silica; the magnetic substance is ferric oxide) included in MagExtractor (registered trade name). The content of the beads in the magnetic bead solution was about 458 mg/mL.

### (Operation 2)

100 µL of human whole blood was added to the liquid exchange vessel 31, and the reagent liquid a and the whole blood were mixed by pipetting for 10 minutes. Through this operation, the nucleic acid is adsorbed onto the surfaces of the magnetic beads. The pipetting speed was set to "1 aspiration discharge / second". Similarly, all of the subsequent pipetting speeds were set to "1 aspiration-discharge / second".

### (Operation 3)

A magnet (permanent magnet) was contacted with the outer wall face of the liquid exchange vessel 31 and moved upwards. Then, the magnet was moved horizontally to the upper part of the adjacent liquid exchange vessel 32, and moved downwards. Thereby, the magnetic beads were moved alone from the liquid exchange vessel 31 to the liquid exchange vessel 32. After confirming the move, the permanent magnet was separated from the outer wall face of the liquid exchange vessel 32. The speed for moving the permanent magnet was set to 30 mm/second. The speeds in all of the subsequent operations were the same.

### (Operation 4)

In the liquid exchange vessel 32, the reagent liquid *b* and the magnetic beads were mixed by pipetting for 10 seconds with a 1mL pipette.

### (Operation 5)

The permanent magnet was contacted with the outer wall face of the liquid exchange vessel 32 and moved upwards. Then, the magnet was moved horizontally to the upper part of the adjacent liquid exchange vessel 33, and moved downwards. Thereby, the magnetic beads were moved alone from the liquid exchange vessel 32 to the liquid exchange vessel 33. After confirming the move, the permanent magnet was separated from the outer wall face of the liquid exchange vessel 33.

### (Operation 6)

In the liquid exchange vessel 33, the reagent liquid c and the magnetic beads were mixed by pipetting for 10 seconds with a 1 mL pipette that is different from the pipette in the above Operation 4.

### (Operation 7)

The permanent magnet was contacted with the outer wall face of the liquid exchange vessel 33 and moved upwards. Then, the magnet was moved horizontally to the upper part of the adjacent liquid exchange vessel 34, and moved downwards. Thereby, the magnetic beads were moved alone from the liquid exchange vessel 33 to the liquid exchange vessel 34. After confirming the move, the permanent magnet was separated from the outer wall face of the liquid exchange vessel 34.

### (Operation 8)

In the liquid exchange vessel 34, the reagent liquid *d* and the magnetic beads were mixed by pipetting for 10 seconds with a 1 mL pipette that is different from the pipettes in the above Operation 4 and Operation 6.

### (Operation 9)

The permanent magnet was contacted with the outer wall face of the liquid exchange vessel 34 and moved upwards. Then, the magnet was moved horizontally to the upper part of the adjacent liquid exchange vessel 35, and moved downwards. Thereby, the magnetic beads were moved alone from the liquid exchange vessel 34 to the liquid exchange vessel 35. After confirming the move, the permanent magnet was separated from the outer wall face of the liquid exchange vessel 35.

### (Operation 10)

In the liquid exchange vessel 35, the reagent liquid e (extraction solution (elution liquid)) and the magnetic beads were mixed by pipetting for 10 minutes with a 100 µL pipet.

### (Operation 11)

The permanent magnet was contacted with the outer wall face of the liquid exchange vessel 35 and moved upwards to the upper part of the empty liquid exchange vessel 34 and then moved downwards. Thereby, the magnetic beads were moved alone from the liquid exchange vessel 35 to the liquid exchange vessel 34, and only the nucleic acid solution separated from the magnetic beads and purified was present in the liquid exchange vessel 35.

### (Operation 12)

The purified nucleic acid solution in the liquid exchange vessel 35 was transferred to a cell of a spectrophotometer for performing spectrophotometry.

### (Comparative Example 1)

The MagExtractor (registered trade name) manufactured by TOYOBO CO., LTD. was used. Corresponding to the operation guide, a nucleic acid was extracted from 100 µL of blood (whole blood). According to this method, the reagent liquids were exchanged in a state where a magnet was disposed at the exterior of the bottom of a microtube so as to hold and fix the magnetic beads at the bottom of the microtube. For the thus obtained extraction solution, the nucleic acid purification degree and the nucleic acid concentration (recovery) were calculated in the same manner as in Example 1. The results are shown in Table 1 below and the graph of FIG. 8.

**[Table 1]**

| | 260 nm absorbance (A) | 280 nm absorbance (B) | Purification degree (A/B) | Concentration (ng/µL) |
|---|---|---|---|---|
| Example 1 | 0.51 | 0.28 | 1.86 | 26 |
| Comparative Example 1 | 0.44 | 0.24 | 1.85 | 22 |

As shown in Table 1 and the graph of FIG. 8, the nucleic acid purification degrees were substantially the same and excellent in Example 1 and Comparative Example 1. For the recovery, however, Example 1 was superior by about 15% to Comparative Example 1. Moreover in Example 1, a phenomenon in which the magnetic beads remain in the liquid exchange vessel at the time of the move of the magnetic beads (i.e., the loss of magnetic beads) did not occur. At contrast, in Comparative Example 1, the loss of magnetic beads occurred. There was a great difference in the recovery between Example 1 of the present invention and Comparative Example 1 representing a conventional technique. The reason is considered as follows. That is, according to Example 1 of the present invention, there is substantially no loss in the magnetic beads, and at the time of the move of the magnetic beads, only the magnetic beads can be moved with very little amount of reagent liquid.

### Industrial Applicability

As mentioned above, according to the liquid exchange method of the present invention, a remaining liquid will not be included in a subsequent step. Therefore, for example, in extraction of an ingredient from a sample, the extraction accuracy is excellent and a loss of the magnetic substance can be prevented. Moreover, even when the liquid exchange method of the present invention is applied to an autoanalyzer, the device structure will not be complicated. Therefore, the liquid exchange method of the present invention can be applied to all fields where a liquid present around a magnetic substance must be exchanged, and for example, the method of the present invention can be applied preferably to extraction of an ingredient from a sample of biological origin, though the application is not limited thereto.

## Claims

1. A liquid exchange method for exchanging liquids present around a magnetic substance, the method comprising:
providing a plurality of vessels containing liquids, a magnetic substance move-mediating member that mediates a move of the magnetic substance from one of the vessels to another vessel, and a magnet,
wherein the liquid in at least one of the vessels is a magnetic substance containing liquid;
disposing the magnet at the exterior of the vessel;
moving the magnet so as to move the magnetic substance in the magnetic substance containing liquid on the inner wall face of the vessel in order to take out the magnetic substance from the magnetic substance containing liquid;
moving the magnet so as to move the magnetic substance from the inner wall face of the vessel to a surface of the magnetic substance move-mediating member; and
moving the magnet so as to move the magnetic substance to an inner wall face of another vessel via the surface of the magnetic substance move-mediating member in order to introduce the magnetic substance into the liquid in the other vessel, thereby exchanging the liquids present around the magnetic substance.

2. The liquid exchange method according to claim 1, wherein the magnet is moved to move the magnetic substance into the liquid on the inner wall face of the other vessel.

3. The liquid exchange method according to claim 1 or 2, wherein the magnet is moved further to move the magnetic substance on the inner wall face of the other vessel in order to take out the magnetic substance from the magnetic substance containing liquid, and the magnet is moved to move the magnetic substance from the inner wall face of the other vessel to the surface of the magnetic substance move-mediating member,
the magnet is moved to move the magnetic substance further onto an inner wall face of still another (third) vessel via the surface of the magnetic substance move-mediating member in order to introduce the magnetic substance into the liquid in the third vessel.

4. The liquid exchange method according to claim 1, wherein the magnetic substance comprises magnetic fine particles.

5. An ingredient extraction method of extracting an ingredient from a sample, the method comprising:
adsorbing an extraction target ingredient onto a magnetic substance in an adsorption solution;
exchanging the adsorption solution for a washing liquid in this state so as to wash and remove ingredients other than the extraction target ingredient; and
exchanging further the washing liquid for an elution liquid so as to elute the extraction target ingredient in the sample from the magnetic substance;
wherein the exchange of the respective liquids in the ingredient extraction step is carried out by the liquid exchange method according to claim 1.

6. The ingredient extraction method according to claim 5, wherein the extraction target ingredient is a nucleic acid and the sample is of biological origin.

7. A composite container used for the liquid exchange method according to claim 1 or the ingredient extraction method according to claim 5,
wherein the composite container comprises a plurality of vessels and a magnetic substance move-mediating member, and the vessels and the magnetic substance move-mediating member are integrated with each other.

8. The composite container according to claim 7, wherein the composite container further has a reagent vessel containing a reagent liquid.

9. The composite container according to claim 7 or 8, wherein the composite container is a cartridge for an autoanalyzer.

10. An autoanalyzer for analyzing an ingredient in a sample, the autoanalyzer having an ingredient-extracting member for extracting an ingredient from the sample, and the ingredient-extracting member extracts the ingredient by the ingredient extraction method according to claim 5 or 6.

11. The autoanalyzer according to claim 10, further having a magnet moving member, and moving the magnet with the magnet moving member.

12. The autoanalyzer according to claim 10, using the composite container according to claim 7.

13. The autoanalyzer according to claim 12, further having a composite container moving member and moving the composite container with the composite container moving member so as to move the magnetic substance relatively.
